# EUROPEAN PATENT SPECIFICATION

(11) **EP 0 968 160 B1**
(45) Date of publication and mention of the grant of the patent: **16.08.2001**
(21) Application number: 97946272.8
(22) Date of filing: 10.10.1997
(51) Int. Cl.: C07C 5/22, C07C 5/27

(54) **N-OLEFIN SKELETAL ISOMERIZATION PROCESS**
VERFAHREN ZUR GERÜSTISOMERISIERUNG VON N-OLEFINEN
PROCEDE D'ISOMERISATION DE TYPE A SQUELETTE CARBONE DE N-OLEFINES

(30) Priority: 16.10.1996 US 733057
(43) Date of publication of application: 05.01.2000
(73) Proprietor: MOBIL OIL CORPORATION, Fairfax, Virginia 22037-0001 (US)
(72) Inventor: CHOI, Byung, Chang, Cherry Hill, NJ 08003 (US); KLOCKE, Donald, Joseph, Gloucester Township, NJ 08083 (US); LISSY, Daria, Nowakiwska, Glen Mills, PA 19342 (US); MARLER, David, Owen, Deptford, NJ 08096 (US)
(74) Representative: Kador & Partner
(86) International application number: US9718961
(87) International publication number: WO9816488

(56) References cited:
- EP-B- 0 532 646
- US-A- 5 292 980
- US-A- 5 321 194
- US-A- 5 382 743
- US-A- 5 449 851
- US-A- 5 523 511

## Description

This invention relates to a method for the skeletal isomerization of n-olefin-containing, e.g., n-butene-containing, hydrocarbon streams to iso-olefin-rich, e.g., isobutene-rich product streams.

The demand for iso-alkenes has recently increased in response to a greater demand for oxygenated gasoline additives. For example, relatively large amounts of isobutene are required for reaction with methanol or ethanol over an acidic catalyst to produce methyl tert-butyl ether (MTBE) or ethyl tert-butyl ether (ETBE) which is useful as an octane enhancer for unleaded gasolines. Isoamylenes are required for reaction with methanol over an acidic catalyst to produce tert-amyl methyl ether (TAME). With passage of the Clean Air Act in the United States mandating increased gasoline oxygenate content, MTBE, ETBE and TAME have taken on new value as a clean-air additive, even for lower octane gasolines. Lead phasedown of gasolines in Western Europe has further increased the demand for such oxygenates.

An article by J.D. Chase, et al., Oil and Gas Journal, April 9, 1979, discusses the advantages one can achieve by using such materials to enhance gasoline octane. The blending octane values of MTBE when added to a typical unleaded gasoline base fuel are RON = 118, MON = 101, R+M /2 = 109. The blending octane values of TAME when added to a typical unleaded gasoline base fuel are RON = 112, MON = 99, R+M / 2 = 106. Isobutene (or isobutylene) is in particularly high demand as it is reacted with methanol to produce MTBE.

The addition of shape-selective zeolite additives such as ZSM-5 to cracking catalysts, e.g. those used in fluidized catalytic cracking (FCC), is beneficial in producing gasoline boiling range product of increased octane rating. However, increased amounts of olefins result, including n-butenes, creating a need for their conversion to higher value products such as isobutene which can be used to produce MTBE.

Butene exists in four isomers: butene-1, cis-butene-2, its stereo-isomer transbutene-2, and isobutene. Conversions between the butenes-2 is known as geometric isomerization, whereas that between butene-1 and the butenes-2 is known as position isomerization, double-bond migration, or hydrogen-shift isomerization. The aforementioned three isomers are not branched and are known collectively as normal or n-butenes. Conversion of the n-butenes to isobutene, which is a branched isomer, is widely known as skeletal isomerization.

U.S Patent No. 5,449,851 discloses a process for isomerizing linear C₄ to C₁₀ olefins to the corresponding iso-olefins using a catalyst comprising ZSM-35 preferably in combination with a non-acidic binder, such as silica. Whereas this process offers significant improvements over earlier proposals, it is found that, at the high zeolite levels desirable to enhance catalyst activity and hence n-olefin conversion, there is a decrease in the selectivity of the catalyst after regeneration, particularly where the n-olefin is n-butene.

The present invention resides in a process for the conversion of linear olefins to corresponding iso-olefins of the same carbon number, e.g. n-butenes to isobutene, which comprises contacting a linear olefin-containing organic feedstock with a catalyst comprising ZSM-35 and containing alkaline earth metal cations.

Preferably, the cations are alkaline earth metal cations and most preferably are calcium cations.

Preferably, the catalyst comprises a non-acidic binder, such as silica, and preferably the weight ratio of ZSM-35 to binder is at least 1:1.

Preferably, the linear olefin is a C₄ to C₁₀ olefin and most preferably is n-butene.

### Description of the Drawings

Figure 1 is a graph of n-butene conversion against time for three fresh catalysts, Catalyst A comprising 10 wt% HZSM-35 and 90 wt% silica, Catalyst B comprising Natreated 65wt% ZSM-35 and 35 wt% silica, and Catalyst C comprising Ca-treated 65wt% ZSM-35 and 35 wt% silica;
Figure 2 is a graph of iso-butene yield against time for the three catalysts used in Figure 1;
Figure 3 is a graph of n-butene conversion against time for Catalyst C of Figure 1 before and after regeneration; and
Figure 4 is a graph of iso-butene yield against time for Catalyst C of Figure 1 before and after regeneration.

### Detailed Description of the Invention

The present invention provides a process which converts a linear C₄-C₁₀ olefin-containing hydrocarbon feedstream to an iso-olefin rich product at high iso-olefin selectivity by contacting the feedstream under skeletal isomerization conditions with a catalyst comprising ZSM-35 and containing alkaline earth metal cations.

ZSM-35 is more particularly described in U.S. Patent No. 4,016,245. For purposes of the present invention, ZSM-35 is considered to include its isotypes, which include ferrierite (P.A. Vaughan, Acta Cryst. 21, 983 (1966)); FU-9 (D. Seddon and T. V. Whittam, European Patent B-55,529, 1985); ISI-6 (N. Morimoto, K. Takatsu and M. Sugimoto, U.S. Patent 4,578,259, 1986); monoclinic ferrierite (R. Gramlich-Meier, V. Gramlich and W. M. Meier, Am. Mineral. 70, 619 (1985)); NU-23 (T. V. Whittam, European Patent A-103,981, 1984); and Sr-D (R. M. Barrer and D. J. Marshall, J. Chem. Soc. 1964, 2296 (1964)).

The ZSM-35 catalyst used in the process of the invention contains alkaline earth metal cations, preferably calcium cations which can be introduced into the catalyst by conventional impregnation or ion exchange techniques. Preferably the amount of alkaline earth metal cations contained by the catalyst is at least 0 5%, and more preferably at least 1%, by weight of the catalyst but less than the ion exchange capacity, and most preferably less than 50% of the ion exchange capacity, of the ZSM-35.

The cation-containing catalyst preferably has an alpha value less than 100, and more preferably less than 60, when used in the process of the present invention. Alpha value of a zeolite is a measure of zeolite acidic functionality and is more fully described together with details of its measurement in U.S. Patent No. 4,016,218, J. Catalysis, 6, pp. 278-287 (1966) and J. Catalysis, 61, pp. 390-396 (1980). The experimental conditions cited in the latter reference are used for characterizing the catalysts described herein.

The ZSM-35 used in the process of the invention may be combined with another material usually referred to as a matrix or binder. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the subbentonites and the kaolins commonly known as Dixie, McNamee, Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolites employed herein may be composited with a porous matrix material, such as silica, alumina, zirconia, titania, silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. The matrix can be in the form of a cogel. A mixture of these components could also be used.

Of all the foregoing materials, non-acidic binders, such as silica, are preferred as the matrix material owing to their relative inertness for catalytic cracking reactions which are preferably minimized in the instant isomerization processes. The relative proportions of zeolite and binder may vary widely with the zeolite content ranging from about 1 to about 90 percent by weight and more usually in the range of about 30 to about 80 percent by weight of the composite. Preferably, the weight ratio of zeolite to binder is at least 1:1.

The skeletal isomerization reaction of the present invention is carried out at temperatures between 250 and 750°C; weight hourly space velocity based on linear olefin in the feed between 0.5 and 500 WHSV; a linear olefin partial pressure between 12 and 500 kPa. The preferred conditions are temperatures between 325 and 600°C, more preferably between 375 and 550°C, WHSV between 2 and 400, more preferably between 5 and 250; and a linear olefin partial pressure between 30 and 300 kPa, more preferably between 50 and 150 kPa. Under these conditions the conversion of linear olefin, e.g., n-butene, can be at least 10%, preferably at least 25% and more preferably at least 35%. Generally, economically feasible isomerization is maintained at n-olefin conversion levels above 25 wt%, preferably above 30 wt%.

The present invention is especially suited to processes carried out at high linear olefin to iso-olefin selectivity, e.g, at least 75% at relatively low conversion temperatures and high linear olefin partial pressures. Such processes can maintain selectivities of at least 90, 92 or 95% at a conversion temperature less than or equal to 550, 400 or even 350 C, and linear olefin partial pressures above 2 psia (14 kPa), e.g above 5 psia (34 kPa).

Preferred feedstreams include C4 or C4+ hydrocarbon feedstreams. Linear olefins suited to use in the present invention may be derived from a fresh feedstream, preferably comprising n-butenes and/or n-pentenes, or from the effluent of an iso-olefin etherification reactor which employs alkanol and C4 or C4+ hydrocarbon feedstock. Typical hydrocarbon feedstock materials for isomerization reactions according to the present invention include olefinic streams, such as cracking process light gas containing butene isomers in mixture with substantial amounts of paraffins including n-butane and isobutane. The C4 components usually contain a major amount of unsaturated compounds, such as 10-40% isobutene, 20-55% linear butenes, and small amounts of butadiene. Also, C4+ heavier olefinic hydrocarbon streams may be used, e.g., C4 to C10, preferably C4 to C6 olefinic hydrocarbon streams.

The catalyst of the present invention can be readily regenerated without significant reduction in its selectivity for iso-olefin production. Regeneration is typically carried out under oxidative conditions employing procedures known in the art.

The following Examples illustrate the process of the present invention. In the Examples all parts are by weight unless specifically indicated to the contrary.

### Example 1 Synthesis of ZSM-35.

1.18 parts of aluminum sulfate (17.2% Al₂O₃) were added to a solution containing 9.42 parts H₂O and 1.38 parts of 50% NaOH solution in an autoclave. 0.03 parts of ZSM-35 seeds and 3.20 parts of HiSil 233 precipitated silica were added with agitation, followed by 1.0 parts of pyrrolidine.

The resultant reaction mixture had the following composition, in mole ratios:

| | |
|---|---|
| SiO₂/Al₂O₃ | = 21.5 |
| OH⁻/SiO₂ | = 0.11 |
| H₂O/Al₂O₃ | = 13.5 |
| R/Al₂O₃ | = 6.45 |

where R = pyrrolidine. The mix was crystallized at 105°C with stirring for 74 hours. The ZSM-35 product was filtered, washed with deionized water, and dried at 120°C. The chemical composition of the product was, in wt %:

| | |
|---|---|
| SiO₂ | 76.7 |
| Al₂O₃ | 6.4 |
| Na | 0.84 |
| C | 7.26 |
| N | 2.03 |
| Ash @ 100°C | 85.5 |

with a silica/alumina ratio for the product, in moles, of 20.3/l.

### Example 2 (Comparative) Preparation of low-zeolite content catalyst A

Low zeolite content ZSM-35 catalyst, 10% ZSM-35/90% SiO₂, was prepared by dry mixing the as-synthesized ZSM-35 zeolite from Example 1 with precipitated silica. Colloidal silica, in proportion to give 10% ZSM-35/90% SiO₂ in the catalyst, was added to the dry mix. Sufficient water was added to obtain an extrudable mix and the mix was extruded to 1/20 inch quadrulobe shape and dried at 120°C. The extrudate was then exchanged twice with 1N NH₄NO₃ solution (5 ml of solution per gram of catalyst) at room temperature, rinsed with deionized water, dried at 120°C, and calcined in flowing nitrogen for three hours at 538°C. It was again exchanged twice with 1N NH₄NO₃ solution (5 ml of solution per gram of catalyst) at room temperature, rinsed with deionized water, dried at 120°C, and calcined in flowing air for eight hours at 538°C.

The alpha value of this catalyst was 16. The sodium content was 170 ppm Na.

### Example 3

65% ZSM-35/35% SiO₂ catalyst was prepared by dry mixing the as-synthesized ZSM-35 zeolite with precipitated silica. Colloidal silica, in proportion to give 65% ZSM-35/35% SiO₂ in the catalyst, was added to the dry mix. Sufficient water was added to obtain an extrudable mix and the mix was extruded to 1/20 inch quadrulobe shape and dried at 120°C. The extrudate was then exchanged twice with 1N NH₄NO₃ solution (5 ml of solution per gram of catalyst) at room temperature, rinsed with deionized water, dried at 120°C, and calcined in flowing nitrogen for three hours at 538°C. It was again exchanged twice with 1N NH₄NO₃ solution (5 ml of solution per gram of catalyst) at room temperature, rinsed with deionized water, dried at 120°C, and calcined in flowing air for eight hours at 538°C. The alpha value of this catalyst was 88. The sodium content was 50 ppm Na.

### Example 4 (Comparative) Preparation of Catalyst B

The acidity of the catalyst of Example 3 was modified by the addition of sodium. Sodium nitrate was dissolved in water, to give 1% Na (100% solids basis) on the catalyst, added via incipient wetness to the extrudate of Example 3, allowed to stand for four hours, dried at 120°C, and calcined in flowing air for three hours at 538°C. The alpha value of this catalyst was 18 and the sodium content was 0.95% Na.

### Example 5 Preparation of Catalyst C

The acidity of the catalyst of Example 3 was modified by the addition of calcium. Calcium nitrate was dissolved in water, to give 1.4% Ca (100% solids basis) on the catalyst, added via incipient wetness to the extrudate of Example 3, allowed to stand for four hours, dried at 120°C, and calcined in flowing air for three hours at 538°C. The alpha value of this catalyst was 19 and the calcium content was 1.6% Ca.

### Example 6

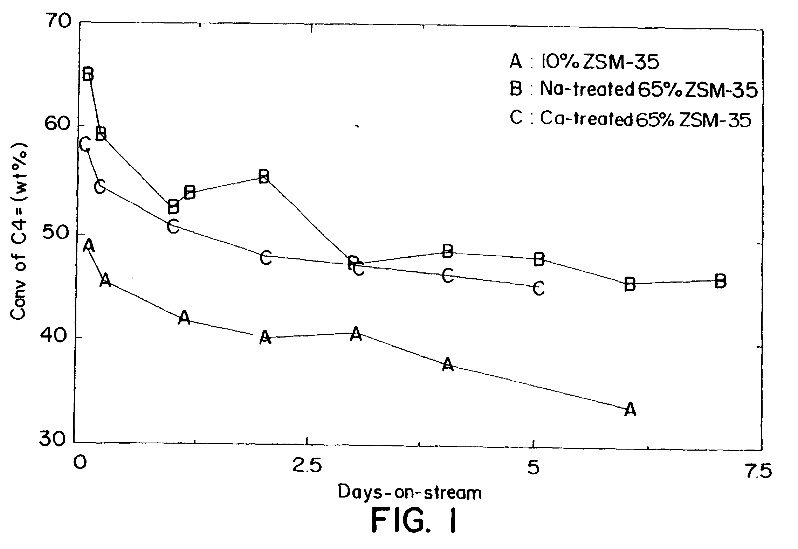

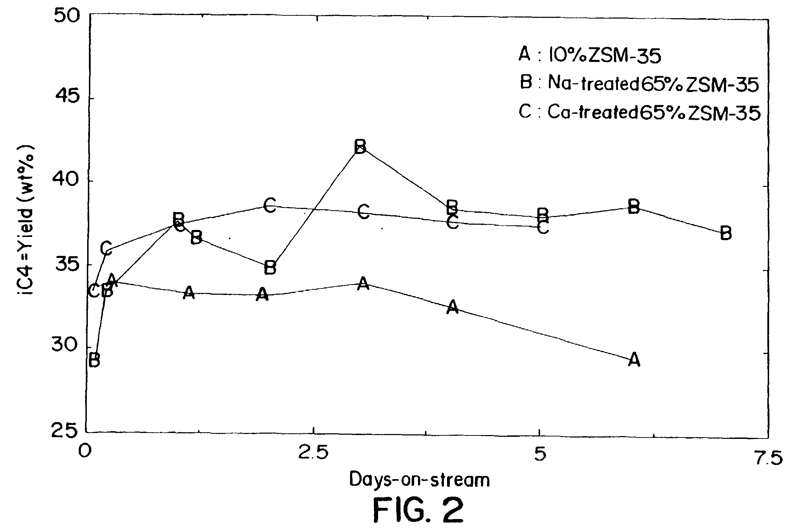

Catalysts A, B and C were tested in the isomerization of n-butene to iso-butene at a temperature of 400°C, a pressure of 15 psig (200 kPa) and a weight hourly space velocity of 7.5 and the results of the tests are shown in Figure 1 and 2. From Figure 1, it will be seen that Catalysts B and C exhibited superior initial activity for the conversion of n-butene than Catalyst A and maintained this enhanced activity over the 7.5 days of the test. Thus Catalysts B and C exhibited initial conversion rates of 60-65 wt% which declined to 45-50 wt%, whereas Catalyst A exhibited an initial conversion rate of 50 wt% which declined to 35 wt%. Figure 2 indicates that the initial iso-butene yield for Catalyst A was the same as that for Catalyst C (33 wt%) and slightly greater than that for Catalyst B (30 wt%). However, the yield of Catalyst A decreased during the test to about 30 wt% whereas the yield of Catalysts B and C rapidly increased to 35-40 wt% and remained at this value throughout the remainder of the test.

### Example 7

Catalyst C was removed from the test unit and regenerated in a tube furnace according to the following procedure:
A gas mixture containing 1% O₂/99% N₂ was passed over the catalyst. The temperature was increased to 232°C and held at that temperature for four hours. It was increased to 454°C and held for sixteen hours. Increased to 540°C and held for four hours. O₂ was increased to 5 vol. % and held for sixteen hours. Finally the temperature was increased to 593°C and held for six hours. At the end of this time, the catalyst was cooled down in the flowing gas.

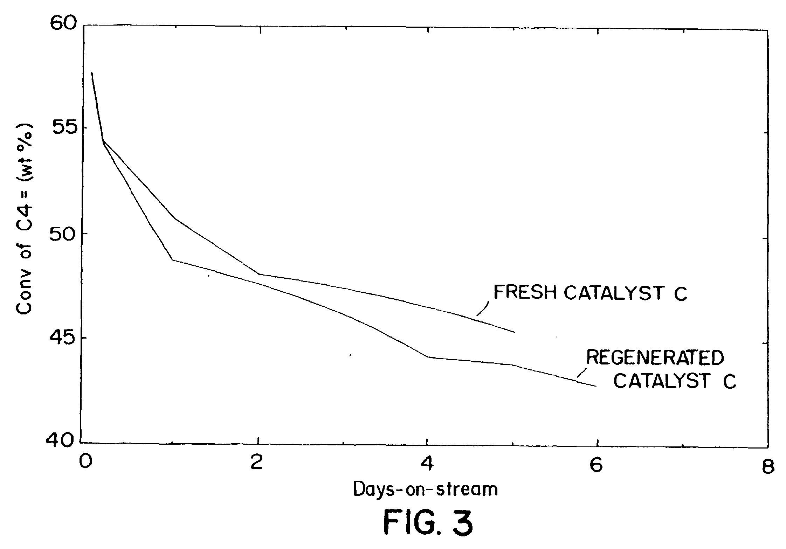

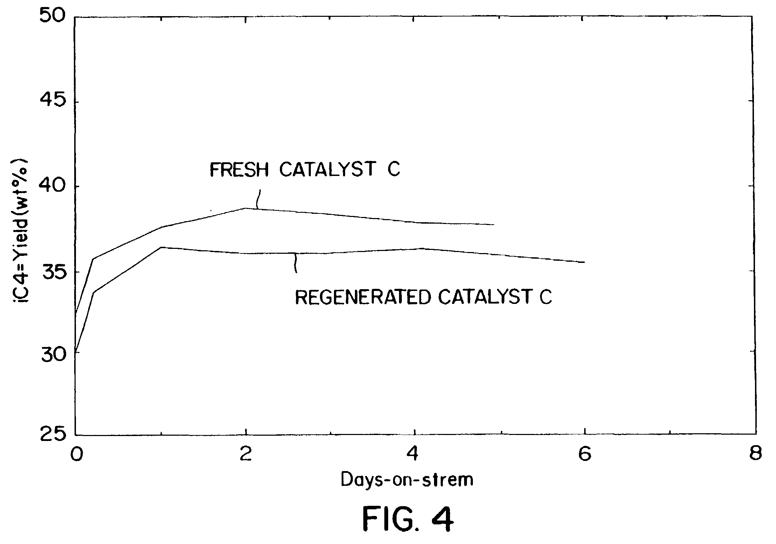

After regeneration Catalyst C was subjected to similar tests to those of Example 6. As shown in Figures 3 and 4, the activity and iso-butene selectivity of the fresh and regenerated catalyst were substantially the same indicating the excellent regenerability of the catalyst of the invention.

Catalyst B was subjected to the same regeneration procedure as Catalyst C but, after regeneration, Catalyst B was found to exhibit low n-butene conversion levels and low yield of iso-butene.

## Claims

1. A process for the conversion of linear olefins to corresponding iso-olefins of the same carbon number which comprises contacting a linear olefin-containing organic feedstock with a catalyst comprising ZSM-35 and containing alkaline earth metal cations.

2. The process of claim 1, wherein the alkaline earth metal is calcium.

3. The process of claim 1, wherein the alkaline earth metal content of the catalyst is more than 0.5 wt% of the catalyst.

4. The process of claim 1, wherein the alkaline earth metal content of the catalyst is less than the ion exchange capacity of the ZSM-35.

5. The process of claim 1, wherein the catalyst comprises a non-acidic binder.

6. The process of claim 5, wherein said binder is silica.

7. The process of claim 5, wherein the weight ratio of ZSM-35 to binder is at least 1:1.

8. The process of claim 1, wherein the catalyst has an alpha value less than 100.

9. The process of claim 1, wherein the catalyst has an alpha value less than 60.

10. The process of claim 1, wherein the linear olefin is a C₄ to C₁₀ olefin.

11. The process of claim 1, wherein the linear olefin is n-butene.

12. The process of claim 1, wherein said contacting is conducted at a temperature between about 250 and about 750°C; a weight hourly space velocity based on linear olefin in the feed between about 0.5 and about 500 and linear olefin partial pressure between about 12 and about 500 kPa.

13. The process of claim 1, wherein said contacting is conducted at a temperature between about 325 and about 600°C, a weight hourly space velocity based on linear olefin in the feed between about 2 and about 400, and a linear olefin partial pressure between about 30 and about 300 kPa.

## Patentansprüche

1. Verfahren zur Umwandlung linearer Olefine in die entsprechenden Isoolefine mit der gleichen Kohlenstoffzahl, das den Kontakt eines lineare Olefine enthaltenden, organischen Beschickungsmaterials mit einem Katalysator umfasst, der ZSM-35 umfasst und Erkalkalimetallkationen enthält.

2. Verfahren nach Anspruch 1, wobei das Erkalkalimetall Calcium ist.

3. Verfahren nach Anspruch 1, wobei der Erkalkalimetallgehalt des Katalysators mehr als 0,5 Gew.-% des Katalysators beträgt.

4. Verfahren nach Anspruch 1, wobei der Erkalkalimetallgehalt des Katalysators geringer als die Ionenaustauschkapazität des ZSM-35 ist.

5. Verfahren nach Anspruch 1, wobei der Katalysator ein nichtsaures Bindemittel umfasst.

6. Verfahren nach Anspruch 5, wobei das Bindemittel Siliciumdioxid ist.

7. Verfahren nach Anspruch 5, wobei das Gewichtsverhältnis von ZSM-35 zum Bindemittel mindestens 1:1 beträgt.

8. Verfahren nach Anspruch 1, wobei der Katalysator einen α-Wert von weniger als 100 hat.

9. Verfahren nach Anspruch 1, wobei der Katalysator einen α-Wert von weniger als 60 hat.

10. Verfahren nach Anspruch 1, wobei das lineare Olefin ein C₄-C₁₀-Olefin ist.

11. Verfahren nach Anspruch 1, wobei das lineare Olefin n-Buten ist.

12. Verfahren nach Anspruch 1, wobei der Kontakt bei einer Temperatur zwischen etwa 250 und etwa 750°C, einer auf das lineare Olefin in der Beschickung bezogenen stündlichen Gewichts-Raum-Geschwindigkeit zwischen etwa 0,5 und etwa 500 und einem Partialdruck des linearen Olefins zwischen etwa 12 und etwa 500 kPa erfolgt.

13. Verfahren nach Anspruch 1, wobei der Kontakt bei einer Temperatur zwischen etwa 325 und etwa 600°C, einer auf das lineare Olefin in der Beschickung bezogenen stündlichen Gewichts-Raum-Geschwindigkeit zwischen etwa 2 und etwa 400 und einem Partialdruck des linearen Olefins zwischen etwa 30 und etwa 300 kPa erfolgt.

## Revendications

1. Procédé de conversion d'oléfines linéaires en iso-oléfines correspondantes ayant le même nombre d'atomes de carbone, selon lequel on met en contact une charge de départ organique à base d'oléfines linéaires, avec un catalyseur contenant de la zéolite ZSM-35 et des cations de métal alcalino-terreux.

2. Procédé selon la revendication 1, dans lequel le métal alcalino-terreux est le calcium.

3. Procédé selon la revendication 1, dans lequel la teneur en métal alcalino-terreux du catalyseur est supérieure à 0,5 % en poids du catalyseur.

4. Procédé selon la revendication 1, dans lequel la teneur en métal alcalino-terreux du catalyseur est inférieure à la capacité d'échange d'ions de la zéolite ZSM-35.

5. Procédé selon la revendication 1, dans lequel le catalyseur comprend un liant non acide.

6. Procédé selon la revendication 5, dans lequel ledit liant est la silice.

7. Procédé selon la revendication 5, dans lequel le rapport pondéral entre zéolite ZSM-35 et liant, est d'au moins 1:1.

8. Procédé selon la revendication 1, dans lequel le catalyseur a un indice alpha inférieur à 100.

9. Procédé selon la revendication 1, dans lequel le catalyseur a un indice alpha inférieur à 60.

10. Procédé selon la revendication 1, dans lequel l'oléfine linéaire est une oléfine en C₄ à C₁₀.

11. Procédé selon la revendication 1, dans lequel l'oléfine linéaire est le n-butène.

12. Procédé selon la revendication 1, dans lequel ladite mise en contact est effectuée à une température d'environ 250 à environ 750 °C ; à une vitesse spatiale horaire pondérale basée sur l'oléfine linéaire dans l'alimentation, d'environ 0,5 à environ 500, et à une pression partielle de l'oléfine linéaire d'environ 12 à environ 500 kPa.

13. Procédé selon la revendication 1, dans lequel ladite mise en contact est effectuée à une température d'environ 325 à environ 600 °C, une vitesse spatiale horaire pondérale basée sur l'oléfine linéaire dans l'alimentation d'environ 2 à environ 400, et une pression partielle d'oléfine linéaire d'environ 30 à environ 300 kPa.
